# EUROPEAN PATENT SPECIFICATION

(11) **EP 1 928 337 B1**
(45) Date of publication and mention of the grant of the patent: **21.11.2012**
(21) Application number: 05790770.1
(22) Date of filing: 29.09.2005
(51) Int. Cl.: A61B 18/18

(54) **APPARATUS FOR TREATMENT OF HOLLOW ORGANS**
VORRICHTUNG ZUR BEHANDLUNG VON HOHLORGANEN
APPAREIL POUR LE TRAITEMENT DES ORGANES CREUX

(43) Date of publication of application: 11.06.2008
(73) Proprietor: Corindus Inc., Natick, MA 01760 (US)
(72) Inventor: BEYAR, Rafael, 34980 Haifa (IL); WENDEROW, Tal, Newton, MA 02465 (US)
(74) Representative: Lawrence, John
(86) International application number: PCT/IL2005/001054
(87) International publication number: WO 2007/036925

(56) References cited:
- EP-A- 0 329 492
- EP-A- 0 590 268
- EP-A- 1 415 660
- EP-A- 1 504 713
- EP-A- 1 554 986
- WO-A-01/74252
- WO-A-02/09571
- US-A- 5 697 377
- US-A- 6 083 170
- US-B1- 6 285 898
- US-B1- 6 447 504

## Description

### FIELD OF THE INVENTION

The present invention relates to apparatuses for treatment of hollow organs. For example, apparatuses are described for performing diagnostic and/or therapeutic procedures in a cardiovascular system. Methods disclosed herein do not form part of the present invention.

### Table 1 Correlations obtained for a given set of samples

Traditionally, people have selected a point of interest in the body and then manually navigated a catheter to that point to perform diagnostic and/or therapeutic procedures. Typically, procedures such as ablation are also performed manually once at the point of interest. Manually navigating to a point of interest and then performing a procedure by hand is time consuming and is not really suitable for hollow organs that move (e.g. the heart). In addition, the procedure itself is inherently dangerous as ofttimes small heart attacks or other cardiac distresses are a byproduct of these procedures. Furthermore, while undergoing these procedures patients are connected to at least a few devices which if any fail, could result in serious injury to the patient. Therefore, concrete benefit could be realized by the patient by reducing the time it takes to perform these procedures. One way to reduce the duration of a procedure is to automate navigation and/or diagnosis and/or therapy. A number of patents describe methods and systems for automatic navigation and/or location of a catheter.

U.S. Patent No. 6,834,201 to Gillies et al. describes a method of magnetically manipulating a medical device within a body part of a human patient in conjunction with MR imaging including applying a navigating magnetic field with magnets from the MR imaging device, and changing the magnetic moment of the medical device to change the orientation of the medical device within the body part.

U.S. Patent No. 6,817,364 to Garibaldi, et al. describes a method of placing a stimulus lead in the heart including introducing a distal end of a delivery catheter into the patient's vasculature; magnetically navigating the distal end of the delivery catheter to the patient's heart; deploying a stimulus lead from the distal end of the delivery catheter; and magnetically navigating the stimulus lead to the stimulus application site.

U.S. Patent No. 6,726,675 to Beyar describes a remote control catheterization system including a propelling device, which controllably inserts a flexible, elongate probe into the body of a patient. A control console, in communication with the propelling device, includes user controls which are operated by a user of the system remote from the patient to control insertion of the probe into the body by the propelling device.

U.S. Patents No. 6,788,967 and 6,690,963 to Ben-Haim, et al. describe a locating system for determining the location and orientation of an invasive medical instrument, for example a catheter or endoscope, relative to a reference frame, comprising: a plurality of field generators which generate known, distinguishable fields, preferably continuous AC magnetic fields, in response to drive signals; a plurality of sensors situated in the invasive medical instrument proximate distal end thereof which generate sensor signals in response to said fields; and a signal processor which has an input for a plurality of signals corresponding to said drive signals and said sensor signals and which produces the three location coordinates and three orientation coordinates of a point on the invasive medical instrument.

A further apparatus is disclosed in EP1415660 A1.

### SUMMARY OF THE INVENTION

The invention is defined in the appended set of claims.

An aspect of some embodiments of the invention relates to reducing the time it takes to perform procedures within hollow organs of a patient body. In an exemplary embodiment of the invention, the time to perform cardiovascular mapping and/or ablating procedures is reduced through the use of an automated navigation and/or mapping and/or ablation system. Various procedures such as electrophysiology ("EP") mapping, pacing the heart and/or ablation are performed in a cardiovascular environment using exemplary embodiments of the invention.

An aspect of some embodiments of the invention relates to automatically issuing commands for navigating procedural instruments to a desired location. Commands include navigation of coaxial movement and/or directional (*i.e*. twisting and/or rotation) components in exemplary embodiments of the invention. Optionally, deflection of a portion (such as the tip) of a procedural instrument is also a command. Optionally, a controller automatically plots a navigational path for the procedural instruments. Using the plotted navigational path, commands are issued to a propulsion device which navigates procedural instruments along that path. Optionally, a navigational path is calculated and followed which optimizes navigation of at least one procedural instrument and/or therapeutic treatment. Optionally, plotted paths and navigation are coordinated with the motion of the hollow organ. Optionally, navigation is performed in or near hollow organs, such as the heart, vascular system, urinary tract, digestive tract, bladder, stomach, intestines and/or gonads. Optionally, navigation is performed in peripheral and/or neuro -interventions. Optionally, a user of the apparatus issues commands to apparatus via the controller; the apparatus then responds to those commands.

An aspect of some embodiments of the invention relates to using EP measurements in addition to, or instead of, position sensing feedback in order to accurately gauge the position of procedural instruments relative to a desired location. EP measurements previously recorded can be related to geographical locations within the arena of operation, using these EP measurements and their correlative geographical position it can be determined where procedural instruments are located within the patient.

An aspect of some embodiments of the invention relates to automatically initiating a therapeutic event at or near a desired location. Using positional information which pinpoints the location of procedural instruments within the operating area in comparison to a map which indicates at least one location which requires therapy, therapeutic events can be initiated automatically by control electronics as the procedural instruments are at or near the locations needing therapy. Optionally, a therapeutic event is pacing, mapping and/or ablation. Optionally, more than one therapeutic event is performed at a particular location.

An aspect of some embodiments of the invention relates to ablating a point, in a line, and/or a more complicated pattern in order to treat a patient. Treatment of heart dysfunction, such as arrhythmia, often requires multiple coordinated ablations in order to provide effective therapy. Using a map of an area of interest which indicates dysfunction, an ablation pattern can be plotted which is likely to be therapeutically effective. Optionally, a pattern of ablation is created without the use of a pre-created map. Optionally, a pattern of ablation is created which optimizes therapy across eso-chrono lines.

A first aspect of the invention provides an apparatus for treating a lumen, comprising: at least one mapping tool, wherein the at least one mapping tool is adapted and constructed to provide at least a partial map of an internal surface of the lumen; a display, adapted and constructed to permit the identification of at least one point of interest in the lumen, using the at least partial map; at least one procedural instrument at least one sensor, wherein data retrieved from the at least one sensor is compared to the at least partial map for determining the position of at least one procedural instrument within the patient; and, a controller wherein controller software is adapted to automatically without human input generate operational commands to the at least one procedural instrument to automatically without human input commence treatment when the determined position of the at least one procedural instrument indicates that at least one procedural instrument is at or near at least one point of interest. Optionally, the apparatus further comprises feedback sensors adapted and constructed for determining the efficacy of the treatment. Optionally, the lumen is a hollow organ. Optionally, the hollow organ is chosen from a group consisting of a heart, a bladder, a brain, a stomach, an intestine or a gonad. The controller identifies the position of the at least one procedural instrument. Optionally, the apparatus further comprises electrophysiology sensors located on the at least one procedural instrument, the sensors in communication with the controller, wherein the sensors assist the controller with the identification of position. Optionally, the controller identifies the position of the at least one procedural instrument using a movement calibration map. Optionally, the apparatus includes an override allowing for manual intervention. Optionally, navigating includes deflecting a tip of the procedural instrument. Optionally, navigating includes advancing of the procedural instrument into the lumen. Optionally, navigating includes retracting of the procedural instrument from the lumen. Optionally, navigating includes rotating the procedural instrument. In some exemplary embodiments of the invention, the apparatus further comprises force sensors adapted to provide data on the amount of force being applied to navigate the at least one procedural instrument. Optionally, the navigation of the at least one procedural instrument ceases upon crossing a predetermined frictional threshold. Optionally, the at least partial map is an EP map. Optionally, the at least partial map is a physiological map.

A method of manipulating a procedural instrument comprises the following steps : automatically without human input mapping at least an internal surface of the lumen, wherein at least a partial map of the lumen internal surface is generated; identifying at least one point of interest in the lumen using the at least partial map; automatically without human input navigating at least one procedural instrument through the lumen using sensors to compare sensed data with the at least partial map. Optionally, the lumen is a hollow organ. Optionally, the at least partial map is an electrophysiology map. Optionally, the at least partial map is a physiological map.

### BRIEF DESCRIPTION OF THE FIGURES

Non-limiting embodiments of the invention will be described with reference to the following description of exemplary embodiments, in conjunction with the Figures. The Figures are generally not shown to scale and any measurements are only meant to be exemplary and not necessarily limiting. In the Figures, identical structures, elements or parts which appear in more than one Figure are preferably labeled with a same or similar number in all the Figures in which they appear, in which:
Fig. 1 is an illustration of a guide catheter and appurtenant instruments, in accordance with an exemplary embodiment of the invention;
Fig. 2 is a schematic depicting the operative relationship between various components of an exemplary catheter use and control system, in accordance with an exemplary embodiment of the invention;
Fig. 3 is a flowchart depicting the navigational process, in accordance with an exemplary embodiment of the invention;
Fig. 4 is a flowchart depicting a method of mapping, in accordance with an exemplary embodiment of the invention;
Fig. 5A is a flowchart depicting a method of ablating, in accordance with an exemplary embodiment of the invention;
Fig. 5B is a flowchart depicting another method of ablating, in accordance with an exemplary embodiment of the invention;
Fig. 6 is an illustration of an ablation pattern, in accordance with an exemplary embodiment of the invention; and
Fig. 7 is a flowchart depicting a method of mapping and ablating using a catheter system, in accordance with an exemplary embodiment of the invention.

### DETAILED DESCRIPTION OF EXEMPLARY EMBODIMENTS

### Overview of Exemplary Instruments Used

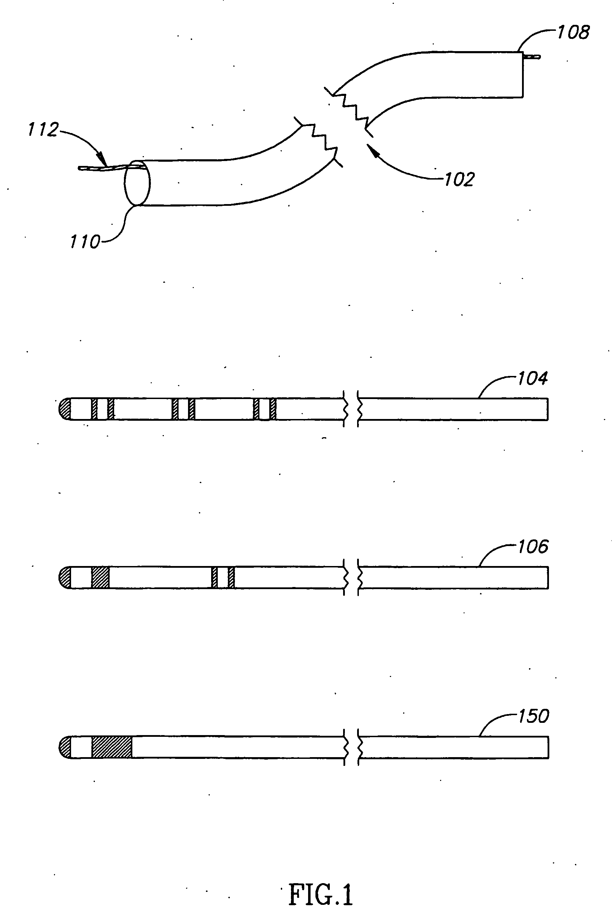

Referring now to Fig. 1, a guide catheter 102 used in an exemplary embodiment of the invention is shown. Optionally, guide catheter 102 is not used.

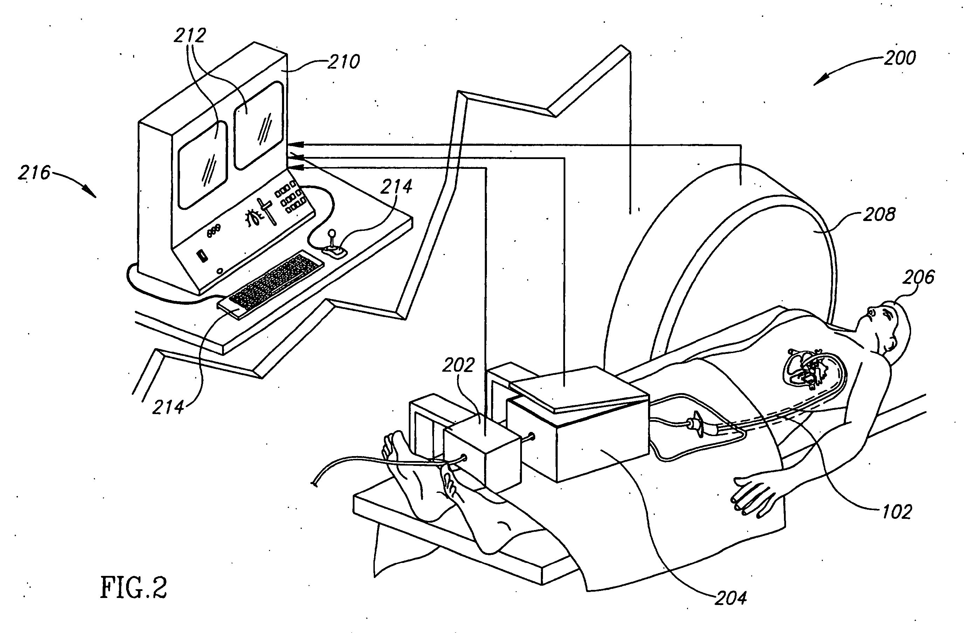

Optionally, guide catheter 102 is used in combination with a mapping 104 and/or ablative 106 instrument and/or pacing instrument 150. Optionally, any or all of the mapping, pacing and ablation functions are performed by the same instrument. In some embodiments of the invention, instruments are inserted through the guide catheter 102 to a desired location within the patient 206, said patient depicted in Fig. 2. A tip which is used for mapping 104 and/or ablating 106 and/or stimulating a desired location is maneuvered to a distal end 108 of the guide catheter 102. In some exemplary embodiments of the invention, instrument operation occurs while in transit in guide catheter 102. Optionally, instruments operate while in transit when there is no guide catheter 102. Optionally, instrument operation while in transit can be used to determine the size of a blood vessel's lumen. The proximal end 110 of the guide catheter 102 is optionally provided with a motion guard which prevents the proximal end 110 of the guide catheter 102 from being propelled out of a propulsion apparatus 202, said apparatus depicted in Fig. 2. Optionally, any of the instruments used with the propulsion apparatus are provided with a motion guard. It should be noted that all devices and/or procedural instruments shown in Fig. 1 are by way of example only, and that other instruments are optionally used in conjunction with the apparatuses and methods described herein, and that other device configurations are optionally used for the devices described herein.

Optionally, the guide catheter 102 and/or mapping instrument 104 and/or the ablative instrument 106 and/or the pacing instrument 150 are utilized in conjunction with a guide wire 112. In an exemplary embodiment of the invention, the guide wire 112 is inserted into the patient and guided to, or past, a desired location and then the guide catheter 102 is placed onto the guide wire 112 and is moved along the guide wire to the desired location. The mapping and/or ablative and/or pacing instruments 104, 106, 150 are then fed through the catheter to or near the desired location where ablation and/or mapping and/or stimulating is performed. While not required, the guide wire 112 can be removed by attending medical personnel once the guide catheter 102 is in position.

Optionally, the distal end of the guide catheter 102 can anchor in a desired location in order to provide additional stability at the distal end of the guide catheter 102. For example, retractable barbs are optionally located at a distal end of guide catheter 102 which lodge into a wall surface near the terminus of the distal end.

### Exemplary System

A schematic depicting the operative relationship between various components of an exemplary catheter use and control system 200 is described by Fig. 2. In an exemplary embodiment of the invention, the system 200 is provided with a catheter propulsion apparatus 202 which is located close enough to a catheter interface 204 that at least a guide catheter 102 can be threaded through each one before insertion into a patient 206. Optionally, the propulsion apparatus 202 is of the type shown and described in U.S. Pat. No. 6,726,675 to Beyar*.*

Propulsion apparatus 202 may be opened for the insertion of the guide catheter 102 and/or other items, such as a mapping instrument 104, a pacing instrument 150, an ablative instrument 106 and/or a guide wire 112. Optionally, other items also include at least one stent and/or at least one angioplasty balloon. Optionally, the above instruments and/or items are automatically fed into propulsion apparatus 202. Optionally, there is separate propulsion for each instrument and device used with the system.

In an exemplary embodiment of the invention, the catheter interface 204 provides access to the guide catheter 102 to assist with various diagnostic and/or therapeutic procedures, such as injection of contrast media and/or any other catheter related procedures known in the art. Also used with the system 200, in an exemplary embodiment of the invention, is at least one fluoroscope 208, which is used to provide images of the patient internally and to show the position of the guide catheter 102, and optionally other instruments, in the patient 206. The system 200 can be used with magnetic resonance imaging and/or a computerized tomography scanning device to observe the procedure being performed on the patient 206, in addition to or in place of a fluoroscope 208. Fluoroscope 208, mapping instrument 104, magnetic resonance imaging and/or computerized tomography scanning devices are optionally considered mapping tools.

In an exemplary embodiment of the invention, various components of the system 200, including at least the propulsion apparatus 202, the catheter interface 204 and the fluoroscope 208 are in operative communication with a controller 210. Optionally or additionally, the controller 210 includes a personal computer. The controller 210 is provided with at least one display device 212 and at least one input apparatus 214, such as a keyboard, mouse, joystick, and/or electronic pad. Optionally, system 200 is used in conjunction with virtual reality devices which provide input and/or output to/from system 200. For example, virtual reality gloves are optionally used to input movement commands to instruments. Another exemplary usage is a virtual reality helmet which displays images to a supervising medical professional. In an exemplary embodiment of the invention, the controller is located in proximity to the patient but in an area 216 shielded from x-rays emitted by the fluoroscope 208. Optionally, the protected area is shielded from other harmful emitted energy, depending on the device used to provide images showing the position of the catheter and/or other instruments being used in the procedure. Optionally, the controller 210 is connected wirelessly to various components of the system 200. In some exemplary embodiments of the invention, the controller 210 is optionally connected to a system adapted for communication to remote locations (in relation to the patient), such as via electrical and/or optical wiring, the telephone system, the Internet and/or other communications technology as is known in the art. Through communication to a remote location, procedures can be performed and/or directed using the system 200 by medical professionals not located in the operating room where the patient is located. Optionally, procedures are performed from remote locations. Furthermore, communication of the procedures being performed to locations outside the operating room allows for collaborative input by medical professionals outside the operating room and allows for using performed procedures for instructional purposes to people located outside the operating room. Various components of the system 200 transmit data to the controller 210 and receive data, including operating instructions, from the controller 210. In some embodiments of the invention, at least one input apparatus is provided with force feedback so that medical personnel operating the system 200 optionally receive tactile information as components of the system progress through a procedure. Optionally, no tactile information is received by medical personnel.

Data received by the controller 210 from various components of the system 200 is displayed on at least one display device 212. Optionally, the data received is processed by the controller 210 and/or stored. Optionally, data received includes: location of guide catheter 102 and other devices inserted into the patient, such as a mapping instrument 104, an ablative instrument 106, a stent, an angioplasty balloon, and/or guide wire 112; information relating to insertion, positioning and movement of the guide catheter 102 and other devices described herein and/or known to those skilled in the art; images gathered by the fluoroscope and/or similar scanning device; status information of propulsion apparatus 202; status information of catheter interface 204; status information of scanning device 208; patient information (e.g. heart rate); data generated by the mapping instrument 104; and/or data generated by the ablative instrument 106.

### Providing Movement

Navigating a catheter and other procedural instruments through the patient and to an area of interest is a delicate and time consuming process. Traditionally, transit was accomplished by a medical professional who manually maneuvered procedural instruments through the patient. In exemplary embodiments of the invention, motive force is provided not by hand, but by a system which supplies automated motion, such as the system described herein. In some embodiments of the invention, the system is controlled by a medical professional using data supplied by the system to perform the procedure, such as described in U.S. Pat. No. 6,726,675 to Beyar. The system is automated, providing movement to the various procedural instruments being used without the need for human intervention, but optionally controlled by an attending medical professional.

In an exemplary embodiment of the invention, three basic types of manipulation and/or motion need to be provided to the instruments in order to navigate the instruments for maximum therapeutic effect. First, the instruments need to be advanced into, and retracted out of, the patient. Second, the instruments often need to be rotated in order to provide more precise control to the instruments themselves. Precision control using rotation is usually facilitated by providing variable angulation to the tip portion of the distal end of the instrument, as is known to those skilled in the art. A third optional type of motion involves deflection of a tip of an instrument. For example, ablation catheters are often provided with deflection capability. Optionally, some or all of these types of manipulation and/or motion are used in conjunction to navigate the procedural instruments. Optionally, one mechanism of propulsion apparatus 202 provides at least one of advancing, retracting, rotating and deflecting a tip of a procedural instrument. Optionally, a plurality of mechanisms of propulsion apparatus 202 provides at least one of advancing, retracting, rotating and deflecting a tip of a procedural instrument.

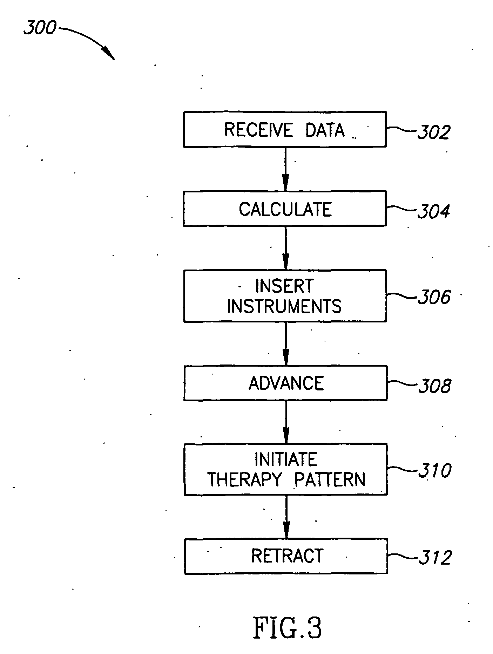

Utilizing the system 200 to perform therapeutic procedures while under the control of a medical professional is described in U.S. Pat. No. 6,726,675 to Beyar*.* However, in some embodiments of the invention, automated control can be harnessed to provide more effective and safer therapeutic results. As described above, the controller 210 receives data from multiple sources which it uses to construct an overall therapy profile for the patient being treated. This action is optionally performed at action 302 of the flowchart 300 depicted in Fig. 3. The therapy profile optionally includes such things as a navigation plan and instrument activation details. In some embodiments of the invention, the therapy profile is calculated at action 304 by pre-programmed software. Optionally, the therapy profile is input into the system by a medical professional. In some embodiments of the invention, the instrument activation details and/or the navigational plan are not pre-programmed but are created real-time by the controller and/or attending medical professional as the procedure takes place.

After instruments are inserted into the patient 206 at action 306, the system 200 begins to advance, at action 308, at least one instrument through the patient and towards an area where therapeutic procedures are to occur. Position identification of instruments relative to the patient and maneuvering to specific positions within the patient are described below. Optionally, a guide wire 112 and/or a guide catheter 102 are used to facilitate transit of instruments through the patient, as described above. Instruments are physically advanced and rotated, within safe parameters, through the patient by the propulsion apparatus 202 of the system 200. As advancement takes place, the force being applied to move the instruments is measured at the propulsion apparatus and conveyed to the controller 210 to ensure that certain proscribed limits are not exceeded. Optionally, propulsion apparatus 202 components are so constructed as to be incapable of propelling instruments further into the patient if the resistance to motion exceeds a certain threshold. For example, a certain frictional threshold is established between propulsion apparatus 202 and an instrument. Below the threshold, propulsion apparatus 202 has propulsion command over the instrument. Above the threshold, propulsion apparatus 202 doesn't have enough hold on the instrument to influence its forward movement into the patient. The frictional threshold is designed to be overcome if an undesirable resistance to motion surpasses the threshold. In such an exemplary embodiment, propulsion apparatus 202 continues to attempt propulsion, but the instrument no longer advances because the threshold has been overcome. Optionally, a medical professional can intervene at any point in the automated process and assume control of the procedure.

Movement of an instrument can be coordinated with external factors, such as the contraction rhythm of the patient's heart. Precise movement can be difficult to achieve while operating in an environment that is periodically in motion. In an exemplary heart scenario, using sensors to detect electrical activity within the heart allows for the controller to estimate when the heart will beat, thereby altering the relative position of an instrument in the heart, if only for a moment. Movement of an instrument in the heart can be coordinated to the activity of the heart by using the calculation of the contraction time. Coordinating movement of an instrument with the movement of the heart has a number of advantages, including the minimization of slippage (*i.e.* unwanted instrument movement, which occurs during contraction) and the potential avoidance of making unintentional contact with the wall of the heart.

Once an instrument is in an area where therapeutic procedures are to be performed, the controller 210 instructs the propulsion apparatus 202 to navigate the instrument in accordance with the therapy profile at action 310. Comparing the position of the instrument with a map of the area where therapy is to be delivered dictates what movement and/or rotation needs to be imparted to the instrument by the system 200. In an exemplary embodiment of the invention, navigation is optimized by calculating a navigational path which performs the most effective therapy with the least amount of movement and/or rotation. Various patterns of therapy that can be delivered are described in more detail below. Optionally, movement of the instrument is random within certain proscribed confines. Optionally, more than one instrument, such as those described herein, is navigated by the system 200.

At action 312, the instrument can be retracted from the patient upon the conclusion of the therapeutic procedure.

### Position Identification

In order to effectively perform therapeutic procedures within a patient, it is extremely important to know where instruments are in relation to the patient. Traditionally, a fluoroscope is used to show, in real time, the patient internally and the location of any instruments within the patent. Therapy is thus conventionally performed using the fluoroscope image possibly overlaid by a map showing the area where therapy, such as ablation, is to be conducted.

There are two basic methods of position tracking within the patient, the first is geographical (*i.e.* physical) and the second is electrical in nature. Both methods can be used separately, or in conjunction, to facilitate effective therapeutic treatment of a patient. As described above, a fluoroscope is optionally used to provide a physical image of the relationship between an instrument and the patient. Additionally or alternatively position sensors, which are known to those skilled in the art, located on instruments can be used to indicate the position of the instruments in relation to a geographical map of the patient. Additionally or alternatively to the controller, a medical professional matches the position sensor readings to the geographic map.

Another method for position tracking involves comparing a map of electrical activity within the patient to readings made by sensors located on procedural instruments. In an exemplary embodiment of the invention, EP sensors are located on the instruments being used within the patient. As they measure the electrical activity around them, these measurements are compared to a map of the EP activity of the patient. Matching the EP map to the instruments' EP readings establishes a position of the instruments relative to the patient. This method is optionally used with, or instead of, geographic position tracking methods. Additionally or alternatively to the controller, a medical professional matches the EP readings to the EP map.

In some embodiments of the invention, the navigational plan relies on a movement calibration map that was previously generated, likely during a prior procedure in the patient. A movement calibration map essentially plots where an object, such as a catheter, moves to within the patient when moved a certain measured amount. Each patient's physiology, while substantially similar, contains certain variations which effect navigation therethrough. In practice, if a movement calibration map is already generated, the position of an instrument is known based simply on the measurement of the various movements performed by the propulsion apparatus 202. For example; if a catheter is advanced 30 cm into the patient by the propulsion apparatus 202, then the resultant position of the catheter within the patient is known precisely by comparing the data from the propulsion apparatus 202 to the movement calibration map. Use of a movement calibration map optionally allows for "dead reckoning" positioning during procedures.

Optionally, a movement calibration map is created by the controller 210 while a current procedure is being conducted. Using position determination methods such as those described herein, the position of an instrument is correlated to specific measured amounts of movement and/or rotation imparted to the instrument by the propulsion apparatus 202. In some embodiments of the invention, the movement calibration map factors in slippage and/or other unintentional movement of the instrument due to, for example, heart palpitations.

In an exemplary embodiment of the invention, position identification is desired in the context of the heart. For example, it is highly desirable to know the position of therapeutic instruments in relation to the sinoatrial ("s-a") and atrioventricular ("a-v") nodes of the right atrium. Optionally, geographical and/or electrical position sensing methods are used to gauge instrument position within this area. Optionally, instrument positioning is determined relative to the eso-chrono lines of the heart.

### Automatically Initiating Therapy

In an exemplary embodiment of the invention, automatic navigation and position sensing are combined with automatic instrument activation. Once navigation and position sensing are combined to precisely maneuver procedural instruments into an area where therapy is to be delivered, automatic instrument activation is used to render therapy to the patient. Therapy optionally includes mapping, pacing and/or ablation. Using at least one of the three automated methods with the present inventive system can improve the safety of the procedure as well as operating conditions for the attending medical professional.

### Mapping

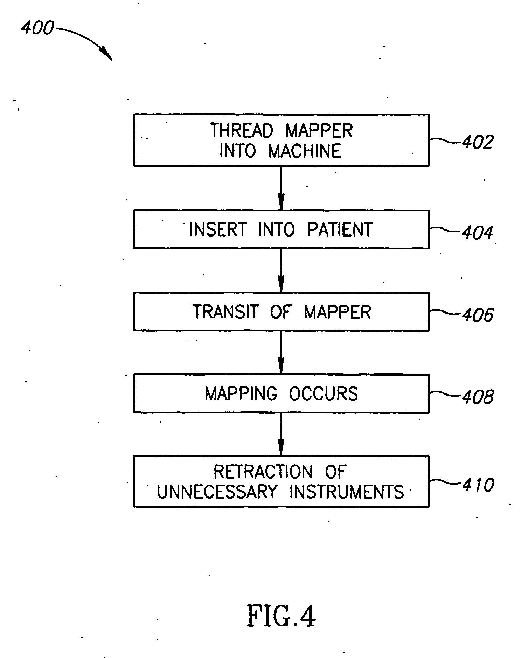

In an exemplary embodiment of the invention, operating instructions the controller 210 issues include instructions to at least a mapping instrument 104. The mapping instrument 104 and the following method are used to sense abnormalities in the heart and/or vascular system in the patient which can then be treated, such as by the ablation technique described herein and/or by techniques known in the art. Turning now to Fig. 4, a flowchart 400 describing a procedure for mapping in the heart and/or vascular system is depicted. At action 402, at least a mapping instrument 104 is threaded into a propulsion apparatus 202. In some exemplary embodiments of the invention, a pacing instrument 150 is also inserted into the propulsion apparatus 202 which is used for instigating a heart arrhythmia. An electric stimulus delivered by the pacing instrument 150 can be used to instigate a heart arrhythmia in some embodiments of the invention. In some exemplary embodiments of the invention, the instruments are also threaded through a catheter interface 204. Optionally, the mapping instrument 104 is capable of instigating a heart arrhythmia (*i.e*. pacing) as well as sensing heart abnormalities. Upon preparation of the patient, the instruments are inserted into the patient at action 404. In an exemplary embodiment of the invention, the threading at action 402 occurs contemporaneously or after the patient is prepared for the procedure. Optionally, the instruments are inserted with the assistance of a guide wire 112 and/or a guide catheter 102. In an exemplary embodiment of the invention, the motive force for insertion is supplied by the propulsion apparatus 202. Alternatively or in addition to the propulsion apparatus 202, motive force is supplied by an attending medical professional.

At action 406, the inserted instruments transit through the patient's vascular system to a desired location within the patient. In an exemplary embodiment of the invention, transit is facilitated by the propulsion apparatus 202 which is in turn operated by a medical professional located at or near the controller 210. Optionally, a medical professional conducts the procedure from a remote location via a communications network, such as the Internet. The propulsion apparatus is commanded by software in communication with the controller 210 without human input (*i.e.* the process is automated), as described above. Transit of the instruments is precisely controlled by the controller 210 and the propulsion apparatus 202, allowing for forward and backward motion with respect to the path of transit. In addition to forward and backward motion, the propulsion apparatus 202 is equipped to provide rotational motion to any of the instruments used, individually and/or severally. A propulsion apparatus, such as described in U.S. Pat. No. 6,726,675 to Beyar and offered by Navicath Ltd., would be suitable for this purpose. In some exemplary embodiments of the invention, the instruments are navigated to avoid interference with objects already located inside the patient. Such objects can include stents and/or a guide wire and/or instruments previously inserted into the patient during the procedure.

At action 408, the system 200 is used to map at and/or in the vicinity of a desired location. Using the propulsion apparatus 202 via the controller 210, the system 200 manipulates the necessary instruments to locations where mapping is desired. In an exemplary embodiment of the invention, a pacing instrument 150 is maneuvered, as described herein, to and/or around the vicinity of a desired location and applies a small electric stimulus to the heart tissue in proximity to the instrument 150. The mapping instrument 104 is also maneuvered, by the system and methods described herein, to a vantage point whereby the mapping instrument 104 is in a position to detect the behavior of the stimulated heart tissue. In an exemplary embodiment of the invention, the mapping instrument 104 communicates observed measurements to the controller 210 for processing, display and/or storage. Optionally, the process of maneuvering the instruments around the heart for stimulating and observing is continued until a map is constructed which is suitable for administering further therapeutic procedures, such as ablation. A model of the mapped area is optionally constructed from the data collected by the mapping instrument 104. Optionally, the model is used as a reference source for navigation, position sensing and ablation, as described herein.

In an exemplary embodiment of the invention, the mapping at action 408 is automated. Commands generated by software at the controller 210 are passed to the propulsion apparatus 202 which maneuvers, as described herein, the pacing and mapping instruments in concert in order to generate a map of the surveyed area. Optionally, the propulsion apparatus 202 moves the instruments in a random pattern. Optionally, the pacing and mapping instruments are moved in a more systematic, pre-programmed pattern. In some exemplary embodiments of the invention, electrical measurements of surrounding tissue are made from the point of insertion to the farthest point of travel of the mapping instrument 104 in order to create a positional reference source capable of being used during various procedures in the patient. Measurements made are communicated to the controller 210 for processing, display and/or storage.

At action 410, any instruments not needed for further therapeutic procedures can be retracted from the patient 206.

### Ablation

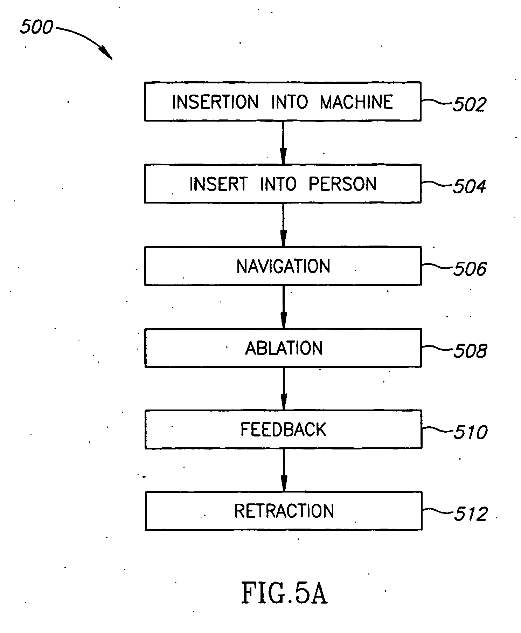

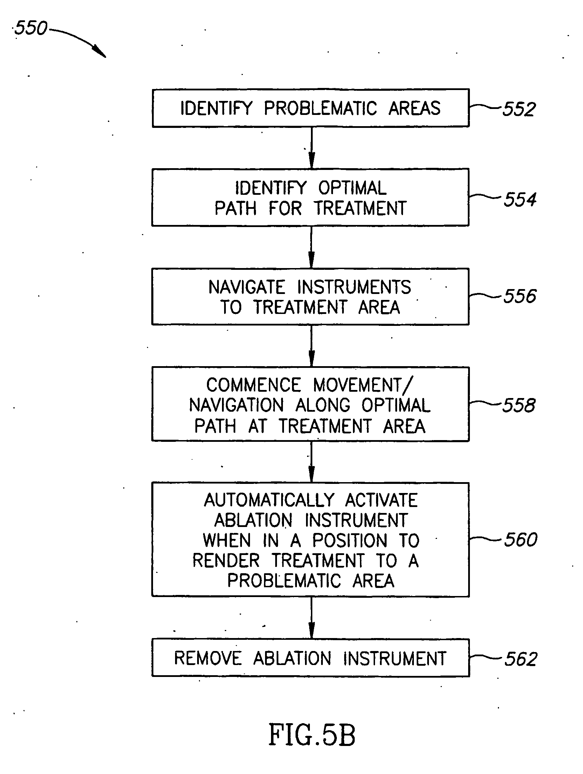

Turning now to Fig. 5, a flowchart 500 depicting a method for ablation therapy is shown. As described herein, ablation is performed in an exemplary embodiment of the invention by an ablation instrument 106 which utilizes RF energy to achieve ablative effect. Optionally, the ablation instrument 106 is non-contact. Optionally, ablation is performed by a laser, toxin, knife, and/or cryogenics. At action 502 the ablation instrument is inserted into the propulsion apparatus 202 and optionally the catheter interface 204. Once the ablation instrument is in a position to be maneuvered by the propulsion apparatus 202, the ablation instrument 106 is inserted into the patient 206 at action 504.

In an exemplary embodiment of the invention, the ablation instrument 106 is navigated, at action 506, towards a desired area. Navigation of the ablation instrument 106 is performed as described above. In an exemplary embodiment of the invention, software is provided which uses the positional sensing methods described above in combination with a reference source to maneuver the ablation instrument 106 into the area where therapy is to take place. Therefore, the ablation instrument 106 is navigated towards a desired area without the assistance of a medical professional.

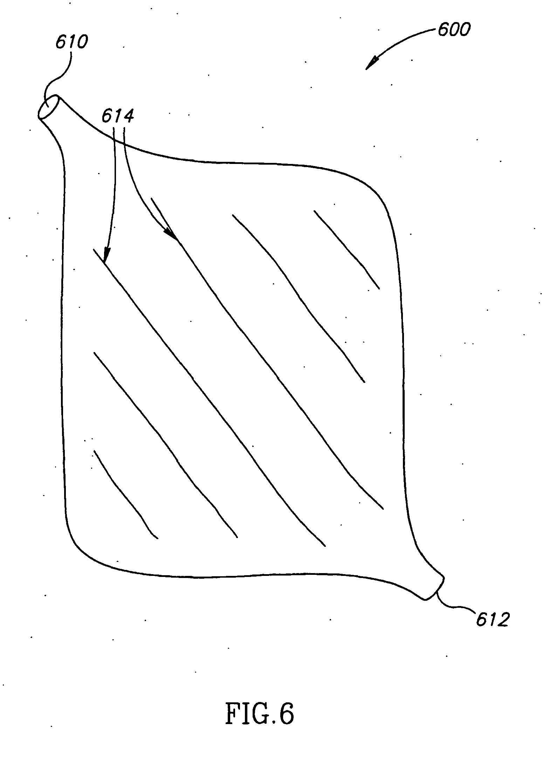

At action 508, therapeutic ablation is performed on sites which are suspected of causing abnormal bodily function. In an exemplary embodiment of the invention, ablation is performed automatically by the system 200. Software is provided on the controller 210 which creates a map, optionally three dimensional, of the area where the procedure is to take place and the problematic areas previously mapped. Using the navigation methods described herein, the system 200 maneuvers the ablation instrument 106 in a manner consistent with rendering therapy at the problematic areas previously mapped. Optionally, the controller 210 calculates a navigational path for rendering therapy to the problematic areas which optimizes motion of the ablation instrument, thereby saving time. The software also tracks the ablation instrument 106, using the positional sensing methods described above, in relation to the problematic areas. In an exemplary embodiment of the invention, software in the controller 210 activates the ablation instrument 106 when it is in an appropriate position to provide therapy without depending on a medical professional for instrument 106 activation. Appropriate position also includes correct orientation of the ablation instrument. While some ablation instruments are omni-directional, other instruments require orientation so that the ablative component is immediately adjacent to the area being treated. Using the rotational ability of the propulsion apparatus 202 as described above, proper orientation can be provided by the system 200. Ablation therapy is optionally performed at one specific point, in a continuous line, and/or in a more complicated pattern, depending on the needs of the patient. Optionally, ablation is overlapping. Fig. 6 depicts a pattern of ablation which is performed in the right atrium 600 of the heart. Lines 614 of ablation are delivered from the s-a node 610 to the a-v node 612 in order to provide effective therapy to this patient.

In an exemplary embodiment of the invention, the ablation instrument 106 is navigated by a medical professional via the controller 210 and the propulsion apparatus 202 to problematic areas mapped by the mapping instrument 104. In order to assist the medical professional as the ablation instrument moves towards the problematic areas, a map of these areas is displayed in conjunction with a current map displaying the position of the ablation instrument 106. Optionally, the map of the problematic areas and the ablation instrument positional map are overlaid on top of one another on the display 212. Once medical professional determines the ablation instrument 106 is in a position to ablate the problematic area, the instrument 106 is automatically activated. Optionally, software in the controller 210 provides a prompt to the medical professional that the ablation instrument 106 is in an appropriate position for activation. Upon the completion of an ablation procedure, the instrument 106 is optionally retracted 512 from the patient.

Turning now to Fig. 5B, a method of ablation for treating heart arrhythmia is depicted in a flowchart 550. Using a previously generated map of the patient's heart activity, problematic areas are identified for ablative treatment at action 552. At action 554, the controller 210 identifies an optimal path of motion for the ablation instrument 106 through the previously identified problematic areas. Optionally, the plotted optimal path of motion is a point, a line or a more complex pattern. At action 556; the methods described herein are used by the system 200 for navigating the ablation instrument 106 to the appropriate area in the patient's heart in order to commence the optimal ablation path for treatment. Once on site, the system 200 causes the ablation instrument to be navigated along the previously plotted optimal path at action 558. As described elsewhere herein, navigation is optionally timed to the movement of the heart in order to reduce slippage and to avoid accidental contact between the instrument and the heart. Using the positioning sensing methods described above and comparing them to the map of problematic areas, the system 200 automatically activates, at action 560, the ablation instrument 106 when the instrument is in a position to render effective therapy to the problematic areas. Optionally, ablation instrument 106 is activated less frequently than once every five heartbeats. Optionally, ablation instrument 106 is activated less frequently than once every one heartbeat. Optionally, ablation instrument 106 is activated at least once every heartbeat.

In some embodiments of the invention, activation includes maneuvering the ablation instrument 106 to be in physical contact with the problematic area being treated. As a corollary to that, deactivation involves breaking contact between the instrument and the heart as the instrument moves on to other treatment areas. In an exemplary embodiment of the invention, an attending medical professional is prompted before commencing automatic operation of the system 200. In exemplary embodiments of the invention, ablation occurs at a point, in a line or a more complicated pattern, such as a plurality of parallel lines, see Fig. 6. Upon the conclusion of the optimal path transit, the instrument is optionally removed from the patient at action 562. In an exemplary embodiment of the invention, feedback is used to test the efficacy of the treatment prior to the removal of the ablation instrument. Feedback is described in further detail below, in the "Feedback" section. In an exemplary embodiment of the invention, areas where treatment is deemed to have been not completely successful are optionally revisited for treatment.

In an exemplary embodiment of the invention, treatment is commenced without having first identified problematic areas of the heart. Thus, an optimal path is not plotted because a map of problematic areas has not been previously generated. In such a case, ablation therapy can still be performed, as described below in the "Mapping and Ablation in Combination" section. In some embodiments of the invention, mapping and ablation are conducted by the same instrument. As described herein, mapping is performed concurrently with ablation in an exemplary embodiment of the invention.

### Mapping and Ablation in Combination

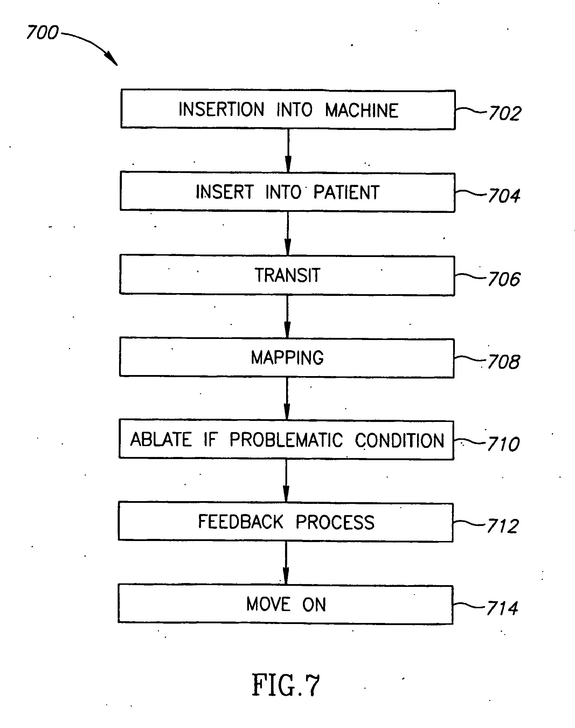

In an exemplary embodiment of the invention, ablation is performed before the mapping process is completed, or optionally, without creating a map. Fig. 7 shows a flowchart 700 depicting a method for using mapping and ablation in combination. At action 702, the instruments needed for mapping and ablation are inserted into the propulsion apparatus 202 and optionally the catheter interface 204. Instruments inserted at action 702 include the mapping instrument 104, the ablation instrument 106 and the pacing instrument 150. In some embodiments of the invention, mapping, ablation and/or pacing are performed by the same instrument. At action 704, the instruments are inserted into the patient. Optionally, insertion is assisted by using a guide wire 112 and/or a guide catheter 102. The instruments are moved into an area of operation as described elsewhere herein, at action 706.

Once in position to diagnose and treat physiological anomalies, the pacing 150 and mapping 104 instruments commence a mapping procedure at action 708, as described above. In an exemplary embodiment of the invention, movement of the instruments is random. Optionally, the instruments follow a pre-programmed motion path constructed to provide comprehensive coverage of the area. Upon the detection of a problematic condition in a specific area, rather than mapping and moving on with the mapping process, the ablation instrument 106 targets the area and administers therapy, at action 710. Before the pacing and mapping instruments move to the next location, at action 712 feedback is optionally gathered from the treated area. Feedback is described in more detail below. If the feedback process detects that the problem has been resolved the instruments move to the next area to be surveyed and/or treated, at action 714. As above, if the problem persists ablation therapy is continued until feedback indicates that therapeutically sufficient levels of ablation have been delivered.

Optionally, this procedure is used if a map has been previously generated during a mapping procedure but the map is of insufficient quality to perform ablation therapy.

### Feedback

The use of ablation to treat dysfunction within a patient is often a hit-or-miss preposition. Due to the risks to the, patient and the time and cost involved with performing catheterization procedures, it is helpful to know the effectiveness of therapeutic procedures while the patient is still "on the table." Therefore, in exemplary embodiments of the invention, feedback is used during and after conducting ablative procedures in order to gauge the effectiveness of those procedures.

At action 510 of Fig. 5 and at action 712 of Fig. 7, feedback is gathered regarding the efficacy of the ablation therapy. Feedback can be microscopic and/or macroscopic. That is, each particular ablation, whether it's a point, a line or a pattern, can be tested for efficacy by making EP measurements around the ablation area. In addition, the overall EP function of the heart can be measured to test the overall efficacy of the course of ablation therapy, especially when more than one ablative event is conducted during the procedure.

In an exemplary embodiment of the invention, feedback is collected contemporaneously with the ablation therapy. Optionally, feedback is collected after a course of ablation therapy. In some embodiments of the invention, feedback entails a procedure similar to the mapping procedure. In order to gauge if the ablation was successful, the heart tissue in the area that was treated is stimulated using the pacing instrument 150. At least the mapping instrument 104 observes the ensuing behavior of the heart tissue. If no abnormalities are sensed, then the ablation therapy that was administered in that area is considered to have been successful. If the problem persists, then another course of ablation therapy is warranted. In an exemplary embodiment of the invention, ablation therapy is continued until feedback indicates that therapeutically sufficient levels of ablation have been delivered. Optionally, feedback is derived from an external system.

Additionally or alternatively, sensors can be located on the instrument which measure EP activity on each side of the ablation area. Ablation is considered successful if the activity on the "downstream" side of the ablative area is significantly reduced. Optionally, using this array of sensors to measure the EP activity around the ablation area factors into the navigation path traveled by the ablative instrument for rendering therapy.

The present invention has been described using detailed descriptions of embodiments thereof that are provided by way of example and are not intended to limit the scope of the invention. The described embodiments comprise different features, not all of which are required in all embodiments of the invention. Some embodiments of the present invention utilize only some of the features or possible combinations of the features. Variations of embodiments of the present invention that are described and embodiments of the present invention comprising different combinations of features noted in the described embodiments will occur to persons of the art. When used in the following claims, the terms "comprises", "includes", "have" and their conjugates mean "including but not limited to". It should also be noted that the device is suitable for both males and female, with male pronouns being used for convenience. The scope of the invention is limited only by the following claims.

## Claims

1. An apparatus for treating a lumen comprising:
at least one mapping tool (104), wherein said at least one mapping tool is adapted and constructed to provide at least a partial map of an internal surface of said lumen;
a display (212) adapted and constructed to permit the identification of at least one point of interest in said lumen, using said at least partial map;
at least one procedural instrument (106,150),
at least one sensor, wherein the apparatus is configured to compare sensed data retrieved from said at least one sensor to said at least partial map for determining the position of at least one procedural instrument within the patient;
a controller (210) encoded with controller software adapted to automatically without human input generate operational commands to the at least one procedural instrument (106,150) to automatically without human input commence treatment when the determined position of the at least one procedural instrument indicates that said at least one procedural instrument is at or near at least one point of interest; and
a propulsion apparatus (202) for automatically without human input controllably navigating said at least one procedural instrument (106,150) in response to operational commands from said controller (210), wherein the apparatus is configured to cease said navigation of said at least one procedural instrument upon crossing a predetermined frictional threshold between the propulsion apparatus and the procedural instrument.

2. An apparatus according to claim 1, further comprising feedback sensors adapted and constructed for determining the efficacy of said treatment.

3. An apparatus according to claim 1, wherein said propulsion apparatus (202) comprises a first mechanism for advancing and retracting said at least one procedural instruments (106,150).

4. An apparatus according to claim 3, wherein said propulsion apparatus (202) comprises a second mechanism for rotating said at least one procedural instrument (106,150).

5. An apparatus according to claim 1, wherein the apparatus is configured to treat a hollow organ.

6. An apparatus according to claim 5, wherein the apparatus is configured to treat a hollow organ_chosen from a group consisting of a heart, a bladder, a brain, a stomach, an intestine or a gonad.

7. An apparatus according to claim 1, wherein said controller (210) is configured to identify the position of said at least one procedural instrument (106,150).

8. An apparatus according to claim 7, wherein the at least one sensor comprises electrophysiology sensors located on said at least one procedural instrument (106,150), said electrophysiology sensors being in communication with said controller (210), wherein said electrophysiology sensors are configured to assist said controller (210) with said identification of position.

9. An apparatus according to claim 1, wherein the controller (210) is configured to identify the position of said at least one procedural instrument (106,150) by comparing navigating movements to a movement calibration map.

10. An apparatus according to claim 1, wherein said propulsion apparatus (202) is configured to deflect a tip of said procedural instrument (106,150) in response to said operational commands.

11. An apparatus according to claim 1, wherein said propulsion apparatus (202) is configured to advance said procedural instrument (106,150) into said lumen in response to said operational commands.

12. An apparatus according to claim 1, wherein said propulsion apparatus (202) is configured to retract said procedural instrument (106,150) from said lumen in response to said operational commands.

13. An apparatus according to claim 1, wherein said propulsion apparatus (202) is configured to rotate said procedural instrument (106,150) in response to said operational commands.

14. An apparatus according to claim 1, wherein said navigating comprises at least one of advancing, retracting, rotating and deflecting a tip of at least one said procedural instruments (106,150).

15. An apparatus according to claim 1, further comprising force sensors adapted to provide data on the amount of force being applied to the at least one procedural instrument to navigate said at least one procedural instrument (106,150).

16. An apparatus according to claim 1, wherein said at least partial map is an electrophysiology map.

17. An apparatus according to claim 1, wherein said at least partial map is a physiological map.

## Patentansprüche

1. Eine Vorrichtung zur Behandlung eines Lumens bestehend aus:
zumindest einem Mapping-Tool (104), wobei das zumindest eine Mapping-Tool so adaptiert und konstruiert ist, dass es zumindest eine teilweise Abbildung einer Innenfläche des genannten Lumens liefert;
einem Display (212), das so adaptiert und konstruiert ist, dass es unter Verwendung der genannten zumindest teilweisen Abbildung die Identifizierung von zumindest einer Stelle von Interesse im genannten Lumen ermöglicht;
zumindest einem Operationsinstrument (106, 150);
zumindest einem Sensor, wobei die Vorrichtung so konfiguriert ist, dass sie erfasste Daten, die vom genannten, zumindest einem Sensor zur genannten zumindest einen Abbildung abgerufen werden, zur Bestimmung der Position von zumindest einem Operationsinstrument im Patienten vergleicht;
einer Steuerung (210), die mit Steuerungssoftware codiert ist, die adaptiert ist, dass sie automatisch ohne menschliche Eingabe mit Behandlung beginnt, wenn die bestimmte Position des zumindest einen Operationsinstruments (106,150) anzeigt, das zumindest eine Operationsinstrument am oder in Nähe einer Stelle von Interesse ist; und
einer Antriebsvorrichtung (202) zur automatischen, kontrollierbaren Navigation ohne menschliche Eingabe des genannten zumindest einen Operationsinstruments (106, 150) in Reaktion auf einen Operationsbefehl von der genannten Steuerung (210), wobei die Vorrichtung so konfiguriert ist, dass sie die genannte Navigation des genannten zumindest einen Operationsinstruments einstellt, wenn eine vorbestimmte Reibeschwelle zwischen der Antriebsvorrichtung und dem Operationsinstrument überschritten wird.

2. Eine Vorrichtung entsprechend Anspruch 1, zu der weiterhin Feedback-Sensoren gehören, die so adaptiert und konstruiert sind, dass sie die Wirksamkeit der genannten Behandlung feststellen.

3. Eine Vorrichtung entsprechend Anspruch 1, wobei die genannte Antriebsvorrichtung (202) einen erste Mechanismus zur Vorfahren und Einfahren des genannten zumindest einen Operationsinstruments (106, 150) aufweist.

4. Eine Vorrichtung entsprechend Anspruch 3, wobei die genannte Antriebsvorrichtung (202) einen zweiten Mechanismus zur Rotation des genannten zumindest einen Operationsinstruments (106,150) aufweist.

5. Eine Vorrichtung entsprechend Anspruch 1, wobei die Vorrichtung zur Behandlung eines Hohlorgans konfiguriert ist.

6. Eine Vorrichtung entsprechend Anspruch 5, wobei die Vorrichtung konfiguriert ist, um ein Hohlorgan aus der folgenden Gruppe, nämlich Herz, Blase, Gehirn, Magen, Darm oder Gonade, zu behandeln.

7. Eine Vorrichtung entsprechend Anspruch 1, wobei die genannte Steuerung (210) konfiguriert ist, um die Position des genannten zumindest einen Operationsinstruments (106, 150) zu identifizieren.

8. Eine Vorrichtung entsprechend Anspruch 7, wobei der zumindest eine Sensor elektrophysiologische Sensoren umfasst, die sich am genannten zumindest einen Operationsinstrument (106, 150) befinden, wobei die genannten elektrophysiologische Sensoren in Kommunikation mit der genannten Steuerung (210) sind, wobei die genannten elektrophysiologische Sensoren konfiguriert sind, um die genannte Steuerung (210) bei der genannten Positionsidentifizierung zu unterstützen.

9. Eine Vorrichtung entsprechend Anspruch 1, wobei die Steuerung (210) konfiguriert ist, um die Position des genannten zumindest einen Operationsinstruments (106, 150) durch Vergleich von Navigationsbewegungen mit einer Bewegungskalibrationsabbildung zu identifizieren.

10. Eine Vorrichtung entsprechend Anspruch 1, wobei die genannte Antriebsvorrichtung (202) konfiguriert ist, um eine Spitze des genannten Operationsinstruments (106, 150) in Reaktion auf die genannten Operationsbefehle abzulenken.

11. Eine Vorrichtung entsprechend Anspruch 1, wobei die genannte Antriebsvorrichtung (202) konfiguriert ist, um das genannte Operationsinstrument (106, 150) in Reaktion auf die genannten Operationsbefehle in das genannte Lumen vorzuschieben.

12. Eine Vorrichtung entsprechend Anspruch 1, wobei die genannte Antriebsvorrichtung (202) konfiguriert ist, um das genannte Operationsinstrument (106, 150) in Reaktion auf die genannten Operationsbefehle aus dem genannten Lumen zurückzuziehen.

13. Eine Vorrichtung entsprechend Anspruch 1, wobei die genannte Antriebsvorrichtung (202) konfiguriert ist, um das genannte Operationsinstrument (106, 150) in Reaktion auf die genannten Operationsbefehle zu rotieren.

14. Eine Vorrichtung entsprechend Anspruch 1, wobei die genannte Navigation zumindest eines der Folgenden umfasst, nämlich Vorfahren, Einziehen, Rotieren und Ablenken einer Spitze von zumindest einem genannten Operationsinstrument (106, 150).

15. Eine Vorrichtung entsprechend Anspruch 1, zu der weiterhin Kraftsensoren gehören, die adaptiert, um Daten über den Grad der angewandten Kraft auf das zumindest eine Operationsinstrument zur Navigation des zumindest einen Operationsinstruments (106, 150) zu liefern.

16. Eine Vorrichtung entsprechend Anspruch 1, wobei es sich bei der zumindest einen teilweisen Abbildung um eine elektrophysiologische Abbildung handelt.

17. Eine Vorrichtung entsprechend Anspruch 1, wobei es sich bei der zumindest teilweisen Abbildung um eine physiologische Abbildung handelt.

## Revendications

1. Un appareil de traitement d'un lumen comprenant :
au moins un outil de cartographie (104) où ledit au moins un outil de cartographie est adapté et construit de façon à fournir une carte au moins partielle d'une surface interne dudit lumen,
un écran (212) adapté et construit de façon à permettre l'identification d'au moins un point d'intérêt dans ledit lumen au moyen de ladite carte au moins partielle,
au moins un instrument opératoire (106, 150),
au moins un capteur, où l'appareil est configuré de façon à comparer des données captées récupérées à partir dudit au moins un capteur à ladite carte au moins partielle de façon à déterminer la position d'au moins un instrument opératoire à l'intérieur du patient,
un système de commande (210) codé avec un logiciel de système de commande adapté de façon à générer automatiquement sans apport humain des commandes opérationnelles vers le au moins un instrument opératoire (106, 150) de façon à, automatiquement sans apport humain, débuter un traitement lorsque la position déterminée du au moins un instrument opératoire indique que ledit au moins un instrument opératoire est au niveau du ou près d'au moins un point d'intérêt, et
un appareil de propulsion (202) destiné à, automatiquement sans apport humain, faire naviguer de manière contrôlable ledit au moins un instrument opératoire (106, 150) en réponse à des commandes opérationnelles provenant dudit système de commande (210),
où l'appareil est configuré de façon à cesser ladite navigation dudit au moins un instrument opératoire en cas de dépassement d'un seuil de friction prédéterminé entre l'appareil de propulsion et l'instrument opératoire.

2. Un appareil selon la Revendication 1, comprenant en outre des capteurs de rétroaction adaptés et construits de façon à déterminer l'efficacité dudit traitement.

3. Un appareil selon la Revendication 1, où ledit appareil de propulsion (202) comprend un premier mécanisme destiné à faire avancer et reculer ledit au moins un instrument opératoire (106, 150).

4. Un appareil selon la Revendication 3, où ledit appareil de propulsion (202) comprend un deuxième mécanisme destiné à faire pivoter ledit au moins un instrument opératoire (106, 150).

5. Un appareil selon la Revendication 1, où l'appareil est configuré de façon à traiter un organe creux.

6. Un appareil selon la Revendication 5, où l'appareil est configuré de façon à traiter un organe creux choisi dans un groupe se composant d'un coeur, une vessie, un cerveau, un estomac, un intestin ou une gonade.

7. Un appareil selon la Revendication 1, où ledit système de commande (210) est configuré de façon à identifier la position dudit au moins un instrument opératoire (106, 150).

8. Un appareil selon la Revendication 7, où le au moins un capteur comprend des capteurs d'électrophysiologie placés sur ledit au moins un instrument opératoire (106, 150), lesdits capteurs d'électrophysiologie étant en communication avec ledit système de commande (210), où lesdits capteurs d'électrophysiologie sont configurés de façon à assister ledit système de commande (210) dans ladite identification de position.

9. Un appareil selon la Revendication 1, où le système de commande (210) est configuré de façon à identifier la position dudit au moins un instrument opératoire (106, 150) par la comparaison de déplacements de navigation avec une carte de calibrage de déplacement.

10. Un appareil selon la Revendication 1, où ledit appareil de propulsion (202) est configuré de façon à dévier une extrémité dudit instrument opératoire (106, 150) en réponse auxdites commandes opérationnelles.

11. Un appareil selon la Revendication 1, où ledit appareil de propulsion (202) est configuré de façon à faire pénétrer ledit instrument opératoire (106, 150) dans ledit lumen en réponse auxdites commandes opérationnelles.

12. Un appareil selon la Revendication 1, où ledit appareil de propulsion (202) est configuré de façon à retirer ledit instrument opératoire (106, 150) dudit lumen en réponse auxdites commandes opérationnelles.

13. Un appareil selon la Revendication 1, où ledit appareil de propulsion (202) est configuré de façon à faire pivoter ledit instrument opératoire (106, 150) en réponse auxdites commandes opérationnelles.

14. Un appareil selon la Revendication 1, où ladite navigation comprend au moins une opération parmi pénétration, retrait, rotation et déviation d'une extrémité dudit au moins un instrument opératoire (106, 150).

15. Un appareil selon la Revendication 1, comprenant en outre des capteurs de force adaptés de façon à fournir des données relatives à la quantité de force qui est appliquée au moins un instrument opératoire de façon à faire naviguer ledit au moins un instrument opératoire (106, 150).

16. Un appareil selon la Revendication 1, où ladite carte au moins partielle est une carte d'électrophysiologie.

17. Un appareil selon la Revendication 1, où ladite carte au moins partielle est une carte physiologique.
